# EUROPEAN PATENT APPLICATION

(11) **EP 0 730 867 A2**
(43) Date of publication of application: **11.09.1996**
(21) Application number: 96103360.2
(22) Date of filing: 05.03.1996
(51) Int. Cl.: A61K 31/725, A61K 31/715, A61K 31/70, A61K 7/48, A61K 7/06

(54) **Non-animal polyanions for use in dermatology and cosmetics**

(30) Priority: 09.03.1995 IT MI950458
(71) Applicant: RES PHARMA S.r.l., 20123 Milano (IT)
(72) Inventor: Facchini, Agostino, 20056 Trezzo Sull'Adda (MI) (IT)
(74) Representative: Minoja, Fabrizio

(57) **Abstract**

The use of polyanions (sulphated polysaccharides; polydeoxyribonucleic acids) of non-animal origin for dermatological and cosmetic treatment of the skin and for trichological treatment, in particular of alopecia, is described.

## Description

The present invention relates to the dermatological and cosmetic sector, in particular to the use of polyanions of non-animal origin in cosmetic skin treatment and in trichological treatment. The present invention also relates to the use of said polyanions for the manufacture of a medicament useful for the treatment of skin wounds.

The present invention furthermore relates to the use of said polyanions for the manufacture of a medicament useful for the treatment of alopecia.

### Background of the invention

It is known that some polyanions (mucopolysaccharides or glucosaminoglycans and polydeoxyribonucleic acids or DNA) extracted from animal organs have two types of activity in dermatology:
- on the skin in general as restructuring, hydrating and capillary-protective agents promoting microcirculation, and as cicatrizing, anti-inflammatory and anti-cellulitis agents;
- on the hair as trichogenic agents since they are capable of inducing and prolonging the anagen phase and for improving trophism of the hair follicle as well as for combatting alopecia.

This latter function is attributed to the sulphated mucopolysaccharides both of a chondroitin nature (chondroitin sulphate A, choindroitin sulphate C and dermatan sulphate) and of a heparin nature (heparin and heparan sulphate) and to the polydeoxyribonucleic acids.

Hyaluronic acid, which contains no sulphate groups, does not have an action inducing the anagen phase, but, together with the other sulphated mucopolysaccharides, helps determine the other activities useful in dermatology and in trichology in particular.

As regards DNAs, it must be remembered that, like trichogenic substances, they have an action inducing the anagen phase of hair, similar to and complementing that of the sulphated mucopolysaccharides, although they have an altogether different chemical structure. The DNA and their derivatives have, moreover, as phosphate group donors, the capability of increasing the cellular energy level (ATP, cAMP, etc.) which, as known, diminishes in cases of cellular disorder and, in particular, in hair follicle cells which are in a critical condition or which are showing the first signs of alopecia.

In trichology and dermatology the function of these substances is not restricted to the transition of the hair from the telogen to the anagen phase or to a general action on the skin, but it has now been clinically shown that the hair and skin tissues assume improved trophic conditions which can be attributed to the intrinsic functions of these substances, in particular the mucopolysaccharides, which constitute the solid and active portion of the fundamental substance or matrix, namely the medium which contains the cells and which governs the exchanges between the cells themselves and the surrounding environment.

In the therapeutic field for the oral or injection administration, these polyanions of animal origin are used as anti-lipemic, anti-cholesterolemic, anti-thrombosis and anti-coagulant agents (only heparin) for circulation deficiencies and for illnesses due to ageing, as well as, in general, as anti-arteriosclerotic agents.

Their use in the dermatological and cosmetic fields, which is nowadays widespread both for hair and skin, can be attributed not only to their trophic, protective, hydrating, regenerative and reconstitutive actions but also to their perfect proven tolerability and the absence of any form of toxicity.

In some countries, the use of extracts derived from animal organs in general or from certain animals in particular is banned for religious reasons. In the European countries and in America and Japan as well, there is a growing sense of mistrust and hostility, and even disgust, on the part of a considerable number of users concerning the use of extracts of animal origin, in particular in the cosmetics sector.

More recently, the reservations expressed regarding their use for the prospective transmission of diseases has led to the search for new mucopolysaccharides and different active polyanions outside the sphere of higher-category animals (cattle, sheep, pigs, fowl, etc.) commonly used for alimentary purposes.

### Summary of the invention

It has now been discovered that in the plant world it is possible to find substitutes which retain the main dermatological and trichological (anti-baldness) characteristics of mucopolysaccharides and other animal-derived biopolymers without arousing suspicion, mistrust, fear and negative attitudes in consumers.

Therefore the present invention relates firstly to the use of polyanions of non-animal origin and compositions which contain them for the trichological treatment of human beings.

The present invention also relates to the use of said polyanions as active principles for the manufacture of a medicament useful for the treatment of skin wounds. Furthermore the present invention also relates to the use of said polyanions for the manufacture of a medicament useful for the treatment of alopecia.

Polyanions of non-animal origin are understood as being those obtained from plant organisms, microorganisms, in particular bacteria and fungi, as well as from lichen.

According to the invention, polyanions comprise polysaccharides and polydeoxyribonucleic acids (DNA).

In this connection, attention has been focussed on some polysaccharides and/or their sulphate derivatives of vegetal, bacterial and fungine origin as well as those derived from lichen or also chitin derivatives, and on DNA, again of vegetal or microbiological origin, which nevertheless have a trichogenic action and retain the same main dermatological characteristics.

While in general dermatology and in dislipemia, polysaccharides also of non-animal origin have been used, in the case of trichogenesis, animal extracts have been used almost exclusively. As regards the other polyanions, such as DNA, in practice only those of animal origin have been used hitherto.

Surprisingly it has now been found that, in addition to general applications, polyanions (polysaccharides and DNA) of vegetal, microbiological and fungine origin as well as some polysaccharides of different origin, such as chitin and chitosan, have also proved to be active in the trichogenesis.

However, in order for these polyanions, which are mainly of vegetal origin, to have a dermatological and trichogenetic action, it is necessary to ensure the presence of certain characteristics which condition and determine the activity thereof.

The molecular weight is preferably comprised between 5,000 and 100,000 (more preferably between 10,000 and 20,000); below 5,000 in particular the trichogenetic activity decreases proportionally, without however disappearing. Above 100,000, in the case of topical application, this activity is no longer significantly detectable, whereas in the case of intradermal injections local reactions occur such as inflammation and oedema, which is nevertheless followed by induction of the anagen phase.

Evidently the molecular weight affects not only the absorption of the substances considered, but also their effect. When, in fact, there is an excessive reduction in the molecular weight the effectiveness of said substances diminishes, whereas when the molecular weight exceeds certain limits there is less tolerability in the administration by injection and no effectiveness in the topical application.

In the case of polysaccharides, in order to promote and prolong the anagen phase of the hair and hence to use them for alopecia, it is essential for them to contain sulphate groups bonded to both the hydroxyls and to the amino groups.

If there are no SO₃H groups present, the anagen phase, i.e. the growth of the cutaneous adnexa, is not stimulated.

In the case where non-sulphated polysaccharides are used, sulphation must be performed.

By way of a guide, the most advantageous amount of sulphur is between 5 and 20%, more preferably between 6 and 12% w/w without excluding different degrees of sulphur content.

Examples of polysaccharides according to the present invention are as follows:
1) GALACTOMANNANS, such as GUAR GUM and CAROB BEAN EXTRACT, of vegetal origin; compounds from galactose/mannose in different proportions.
2) PECTINS - from fruit; based essentially on D-polygalacturonate/rhamnose and other neutral glucides such as D-galactose, L-arabinose, D-xylose and L-fucose. Methoxyl groups are present in pectins.
3) ALGINATES from marine algae. Polymers derived from D-mannosyl-uronic/L-gulosyl-uronic acids.
4) CARRAGEENINS - from marine algae. Their structure consists of alternating copolymers of B-(1,3)-D-galactose and (1,4)-3,6 anhydro-D or L-galactose. Carrageenins which already naturally contain sulphate groups are classified in four different fractions defined iota, kappa, lambda, mu and nu.
5) FUCOIDAN (or fucoidin) and fucans in general - from marine algae. Fucoidan, which is a polysaccharide composed mainly of fucose and is naturally sulphated, is extracted from marine algae in particular of the Fucus and Laminaria type, in particular fucus vesicolosus. Fucoidan, owing to its molecular weight and degree of sulphation, may also be used directly without hydrolysis. It shows a good dermatological and trichogenetic action combatting the loss of hair. In some cases, a mild hydrolysis can increase its effectiveness.
6) XANTHAN GUM - from microorganisms. This is a heteropolysaccharide produced from a bacterium, Xanthomonas campestris, and composed of D-glucosyl, D-mannosyl and D-glucosuronic acid.
7) AGAR AGAR - from marine algae. This is a polysaccharide consisting of two fractions, gelifying agarose, and a sulphated non-gelifying derivative, agaropectin.
8) DEXTRAN - from microorganisms. Many microorganisms produce dextran; the most widely used is Leuconostoc mesenteroides. All dextrans are composed exclusively of d-D-glucopyranoside units which differ only in terms of the type of branching of the structure and the length of the chain. Dextran in solution form is already used in medicine as a plasma substitute. Dextran sulphate is also known in therapeutic treatment as an anti-coagulant and anti-lipoproteinlipemic agent, but, owing to its very low molecular weights, it also has a trichogenetic action unknown hitherto, while its anti-coagulating activity decreases in an inversely proportional manner with respect to its molecular weight.
9) GUM ARABIC, GUM STERCULIA, GUM TRAGACANTH - of vegetal origin. Many vegetal polysaccharide-based gums, if suitably depolymerised and sulphated, possess the necessary properties for a dermatological and trichogenetic action.
10) STARCH AND CELLULOSE and in general vegetal glucans. These glucose polymers, suitably hydrolysed and sulphated, acquire the dermatological properties which are characteristic of this type of compound.
11) CHITIN AND CHITOSAN - derived both from plants and from other organisms. Chitin and chitosan represent a category of polysaccharides of an entirely special kind: in fact, they are the only polysaccharides of a basic nature owing to the amino groups contained in them.

Chitin and chitosan, which is a mainly deacetylated chitin, are fairly widespread in the plant world, in fungi, in yeasts, as well as in lower-category animals such as invertebrates and arthropoda. These are biopolymers which are fairly similar to cellulose and are used in therapy on account of their haemostatic and vulnerary properties.

These two biopolymers are also known in their sulphated form, being experimentally used as anti-coagulants. The dermatological and trichogenic properties which have now been evidenced have hitherto never been considered in connection with their hydrolysed and sulphated forms.

In addition to the aforementioned polysaccharide derivatives, particular interest has also been generated by some sulphated polysaccharides already used in dermatological treatment for topical use or as anti-lipemic agents for general application.

It was also unexpectedly shown that they are capable of inducing and prolonging the anagen phase of hair and prevent loss of the latter. Of these, in addition to dextran sulphate, it is possible to cite as examples the sulphated polysaccharides extracted from fungine hyphae of the Aspergillus type and called heparinoids on account of the similarity to heparin. Of interest is also the preparation obtained by means of sulphation of polygalacturonic acid.

Another example of a derivative already used in therapy for other purposes is pentosan polysulphate consisting, as regards the proportion of polysaccharides, of B-d-xylopyranose residues.

All the listed polysaccharides derived from the non-animal world, when duly hydrolysed, if necessary, and sulphated, showed to a differing degree dermatological and trichogenic characteristics similar to those of mucopolysaccharides from the animal world. In the case where the polysaccharides already naturally possess an adequate molecular weight and degree of sulphation, no modification is required. Among the most interesting, although not exclusive examples, the following may be mentioned: the derivatives of galactomannans, alginates, carrageenin, fucoidan, and fucans in general, gum xanthan, agar agar, chitin and the other sulphated polysaccharides already used in treatment for functions other than the trichogenetic ones never contemplated hitherto.

Since the considered polyanions tend to naturally have high molecular weights, direct or enzymatic hydrolysis must often be performed in order to achieve the desired molecular weights, using conventional methods which are well-known to the person skilled in the art.

Should this operation result in the loss of SO₃H groups for the sulphated polysaccharides, suitable resulphation must be carried out. This operation is anyhow always necessary when the polysaccharides are not sulphated.

For the polydeoxyribonucleic acids or non-animal DNA according to the present invention, it is necessary to emphasize that a mild hydrolysis must always be carried out, checking both the viscosity and the molecular weight achieved by means of the viscosity and the gel filtration.

It must also be pointed out that only the suitably depolymerised DNA is active on the stimulation of the hair anagen phase. The RNA and its hydrolysis products are, from this point of view, completely inactive.

The other polyanions of vegetal, microbiological and fungine origin, which are dermatologically active and trichogenetic, are the partially depolymerised plant DNA. These polydeoxyribonucleic acids originating from the plant world (higher-category plants, algae, fungi, yeasts or bacteria) perform many of the activities of the aforementioned polysaccharides (although they do not have anti-coagulant action), despite having a structure and chemical composition which is entirely different.

As it is known, they are macromolecules which make up the genetic material of all living cells. This material, once it has been extracted and partially depolymerised, performs within the organisms functions which are different from the original ones, in particular in the dermatological sector, and assumes some significant features which make it suitable for replacing and enhancing the action of the sulphated polysaccharides.

Partially depolymerised vegetal polydeoxyribonucleic acid has the following effects:
- on the skin: hydrating, capillary-protective, micro-circulation promoting, cicatrizing, anti-inflammatory, lipolytic and lipid-mobilizing for localized adiposity;
- on hair: like sulphated polysaccharides, they induce and prolong the anagen phase and moreover improve the energy efficiency (ATP and cAMP) which is impaired in the case of alopecia due to the excess of dihydrotestosterone which forms in the hair follicle through the action of the alphareductase.

As it can be seen compared to the polysaccharides, depolymerised vegetal DNA possesses moreover the capability of re-establishing the cellular energy levels as a possible precursor of the metabolites assigned to the production of energy and cellular exchanges.

For extraction of the DNA of non-animal origin, it is necessary to use tissues in the growth stage and spores again originating from the plant world or in any case from microorganisms. Moreover, in addition to spores, the shoots originating from the seeds of various plants are particularly suitable.

By way of a non-limiting example it possible to mention: maize, wheat and soia shoots. In general all plant embryos in the growth stage are suitable. The choice is based on criteria of costs and performance in the active principle.

The buds of higher-category trees are also suitable for the purpose even though their use is less advantageous from an economic point of view.

The use of seeds germinated in the tetraploid state (e.g. maize) is of major interest since, in terms of weight, they contain much higher amounts of DNA compared to normal seeds.

Classic methods are used for the preparation of vegetal DNA and are based on the following techniques:
- grinding of the fresh (or freeze-stored) tissues;
- extraction with a buffered aqueous solution;
- filtration;
- concentration;
- hydrolysis to the required molecular weights, if desired;
- precipitation with solvents;
- purification of the raw product obtained.

The resulting product is titrated in DNA by means of chromatographic, colorimetric and spectophotometric checks and dosing of the organic phosphorus.

As with vegetal polysaccharides, the activity of vegetal DNA is also optimum at molecular weights of between 10,000 and 100,000. Other molecular weights are also active, but to a less advantageous degree. Sulphated polysaccharides and DNA of non-animal origin generically defined as "vegetal" have been shown to have the following actions:
- anti-oedemic and anti-inflammatory action;
- cicatrizating action;
- normalising the circulation and the resistance of vessels to breakage;
- hydrating action.

To determine the direct and indirect actions on the piliferous system and on hair, the following tests were carried out:
- direct induction of the anagen phase when the hairs are in the inactive phase;
- prolongation of the growth phase of human hair, as shown in a trichogram or phototrichogram and by the tear test;
- standardised count of the hair lost following washing (a significant reduction occurs);
- measurement of the reduction in dandruff and seborrhoea in humans;
- determination, again in humans, of the increase in the energy level of the cells of the hair bulb, which is impaired in the case of alopecia (dosage of ATP and cAMP);
- check as to the appearance of the hair, which improves substantially owing to the greater vitality conferred on the hair in general;
- recording of the odour emitted by the scalp.

This odour, which expresses the degree of harmful formation of lipoperoxides, is inhibited by both the biopolymers obtained as described above.

This anti-oxidising activity also expresses an anti-radical action of the substances which form the object of the present invention.
Examples of formulations with the vegetal biopolymers according to the present invention and which can be defined as vegetal sulphated polysaccharides (VSP) and vegetal polydeoxyribonucleic acids (V/DNA). Obviously VSP and V/DNA can be associated with other active principles and other excipients compatible therewith.
- The VSP doses vary from 0.05 to 5%
- The V/DNA doses vary from 0.5 to 10%

| 1) Lotion for hair and other cutaneous adnexa: VSP | |
|---|---|
| VSP | 0.5 g |
| Alcohol 95° | 15 ml |
| Preservatives | qs |
| H₂O to 100 ml | |

| 2) Lotion for hair and for other cutaneous adnexa: V/DNA | |
|---|---|
| V/DNA | 2.5 g |
| Alcohol 95° | 15 ml |
| Preservatives | qs |
| H₂O to 100 ml | |

| 3) Lotion for hair and other cutaneous adnexa: VSP + V/DNA | |
|---|---|
| VSP | 0.2 g |
| V/DNA | 1.5 g |
| Alcohol 95° | 15 ml |
| Preservatives | qs |
| H₂O to 100 ml | |

| 4) Protective capillary cream for the treatment of cellulitis and varicose veins | |
|---|---|
| VSP | 2 g |
| V/DNA | 5 g |
| O/W self-emulsifying base | 25 g |
| Preservatives | qs |
| H₂O to 100 ml | |

| 5) Specific anti-couperose cream | |
|---|---|
| V/DNA | 8 g |
| O/W self-emulsifying base | 30 g |
| Preservatives | qs |
| H₂O to 100 ml | |

| 6) Dermo-protective, moisturising, face and body cream | |
|---|---|
| VSP | 0.2 g |
| V/DNA | 1 g |
| O/W or W/O self-emulsifying base | 20-40 g |
| Preservatives | qs |
| H₂O to 100 ml | |

| 7) Cicatrizing gel | |
|---|---|
| VSP | 1 g |
| V/DNA | 5 g |
| Carbomer | 1 g |
| Neutralizing agents and preservatives | qs |
| H₂O to 100 ml | |

| 8) Dusting powder for irritated, delicate and sensitive skin | |
|---|---|
| Micronized VSP | 0.5 g |
| Micronized V/DNA | 3 g |
| Talc | qs to 100 g |

| 9) Anti-oedemic and anti-inflammatory cream | |
|---|---|
| VSP | 4 g |
| O/W self-emulsifying base | 20 g |
| H₂O to 100 ml | |

## Claims

1. Use of polyanions of non-animal origin for the cosmetic and dermatological treatment of the skin and for trichological treatment.

2. Use as claimed in Claim 1, wherein said polyanions have a molecular weight between 5,000 and 100,000.

3. Use as claimed in Claim 1 or 2, wherein said polyanions are of vegetal, bacterial, fungine and lichen origin.

4. Use as claimed in Claims 1 to 3, wherein said polyanions are sulphated polysaccharides.

5. Use as claimed in Claim 4, wherein said polysaccharides have a sulphur content between 5 and 20% w/w.

6. Use as claimed in Claims 1 to 3, wherein said polysaccharide is a polydeoxyribonucleic acid.

7. Use as claimed in Claims 1 to 5, wherein said polyanion is mixed with cosmetic vehicles and excipients.

8. Use of polyanions of non-animal origin as the active principles for the manufacture of a medicament useful for the treatment of skin wounds.

9. Use of polyanions of non-animal origin for the treatment of alopecia.

10. Use as claimed in Claim 9, wherein said polyanions have a molecular weight between 5,000 and 100,000.

11. Use as claimed in Claim 9 or Claim 10, wherein said polyanion is a sulphated polysaccharide.

12. Use as claimed in Claim 11, wherein said polysaccharide has a sulphur content between 5 and 20% w/w.

13. Use as claimed in Claim 9 or 10, wherein said polyanion is a polydeoxyribonucleic acid.
